# EUROPÄISCHE PATENTSCHRIFT

(11) **EP 3 912 607 B1**
(45) Veröffentlichungstag und Bekanntmachung des Hinweises auf die Patenterteilung: **13.12.2023**
(21) Anmeldenummer: 21175004.7
(22) Anmeldetag: 20.05.2021
(51) Int. Cl.: A61F 9/008, A61F 9/009

(54) **VERFAHREN ZUM BEREITSTELLEN VON STEUERDATEN FÜR EINEN AUGENCHIRURGISCHEN LASER EINER BEHANDLUNGSVORRICHTUNG**
METHOD FOR PROVIDING CONTROL DATA FOR AN OPHTHALMIC SURGICAL LASER OF A TREATMENT DEVICE
PROCÉDÉ DE FOURNITURE DES DONNÉES DE COMMANDE POUR UN LASER CHIRURGICAL OPHTALMIQUE D'UN DISPOSITIF DE TRAITEMENT

(30) Priorität: 22.05.2020 DE 102020113820
(43) Veröffentlichungstag der Anmeldung: 24.11.2021
(73) Patentinhaber: Schwind Eye-Tech-Solutions GmbH, 63801 Kleinostheim (DE)
(72) Erfinder: ARBA MOSQUERA, Samuel, 63739 Aschaffenburg (DE)
(74) Vertreter: Hofstetter, Schurack & Partner

(56) Entgegenhaltungen:
- DE-A1-102006 053 120
- DE-A1-102008 017 293
- US-A1- 2019 247 225

## Beschreibung

Die vorliegende Erfindung betrifft ein Verfahren zum Bereitstellen von Steuerdaten für einen augenchirurgischen Laser einer Behandlungsvorrichtung für die Abtrennung eines Lentikels. Die Erfindung betrifft außerdem eine Behandlungsvorrichtung mit mindestens einem augenchirurgischen Laser und mindestens einer Steuereinrichtung zum Durchführen des Verfahrens, ein Computerprogramm und ein computerlesbares Medium.

Behandlungsvorrichtungen und Verfahren zur Steuerung von ophthalmologischen Lasern zur Korrektur einer optischen Fehlsichtigkeit sind im Stand der Technik bekannt. Dabei können zum Beispiel ein gepulster Laser und eine Strahlfokussierungseinrichtung so ausgebildet sein, dass Laserstrahlpulse in einem innerhalb des organischen Materials gelegenen Fokus eine Photodisruption bewirken, um einen Lentikel aus der Hornhaut (Kornea) zur Korrektur der Fehlsichtigkeit abzutrennen. Bei der Behandlung mit einer Behandlungsvorrichtung zur Abtrennung eines Lentikels wird das Auge üblicherweise durch ein oder mehrere Kontaktelemente der Behandlungsvorrichtung fixiert. Das Kontaktelement ist hierbei ein starres Element, beispielsweise eine plankonkave Linse, die auf das Auge, insbesondere auf die Hornhaut (Kornea), aufgesetzt wird, damit das Auge bei der Behandlung nicht bewegt wird. Nachteilig bei einem solchen Kontaktelement ist es jedoch, dass eine Form der Hornhaut nicht exakt an das Kontaktelement angeglichen sein kann, wodurch sich die Hornhaut durch das Kontaktelement verformt. Hierdurch kann sich auch die Form des abzutrennenden Lentikels verändern, wodurch eine Behandlung, insbesondere ein erreichter Brechkraftwert, fehlerbehaftet sein kann. Um dies auszugleichen kann ein Benutzer der Behandlungsvorrichtung, zum Beispiel ein Arzt, einen zu korrigierenden Brechkraftwert in der Planung der Behandlung gemäß seinen Erfahrungen anpassen, so dass trotz des Kontaktelements der erreichte Brechkraftwert zur Korrektur der optischen Fehlsichtigkeit passt. Jedoch kann trotz der passenden Brechkraft eine erreichte Lentikelhöhe von einer geplanten Lentikelhöhe abweichen, wodurch beispielsweise mehr Gewebe als nötig aus der Hornhaut entfernt werden kann.

Aus der DE 10 2008 017 293 A1 ist ein Verfahren zum Erzeugen von Steuerdaten für eine augenchirurgische Behandlungsvorrichtung bekannt, welche mittels einer Lasereinrichtung in der Augen-Hornhaut Gewebeschichten trennt, wobei im Betrieb der Lasereinrichtung ein eine Kontaktfläche aufweisendes Kontaktglas die Hornhaut in die Form der Kontaktfläche verformt, wozu die Kontaktfläche zuerst mit einem Kontaktflächenscheitel auf einen Hornhautscheitel gesetzt und dann zur Verformung der Hornhaut gegen diese gedrückt wird. In dem Verfahren werden die Steuerdaten für die Lasereinrichtung so erzeugt, dass sie Koordinaten von in der Hornhaut liegenden Zielpunkten für die Lasereinrichtung vorgeben, und bei der Erzeugung der Zielpunkt-Koordinaten wird die Verformung der Hornhaut berücksichtigt, welche während des Betriebs der Lasereinrichtung bedingt durch das Kontaktglas vorliegt.

Aus der US 2019/0247225 A1 ist eine Vorrichtung zur Erzeugung von Steuerdaten für eine Laservorrichtung zur chirurgischen Korrektur einer Fehlsichtigkeit bekannt. Das Verfahren umfasst ein Ändern einer Krümmung eines Augenhintergrundes durch Erzeugung eines Schnitts in der Hornhaut durch Einstrahlen von Laserstrahlung in die Hornhaut, wobei der Schnitt ein Volumen von Hornhautgewebe im Inneren der Hornhaut umschließt und isoliert, und ein Entfernen des umschlossenen Volumens von Hornhautgewebe aus dem Inneren der Hornhaut. Des Weiteren umfasst das Verfahren ein Herstellen des Schnitts, der das Volumen umschließt und isoliert, um einen vorderen und einen hinteren Teil zu umfassen, wobei der vordere Teil mit einem konstanten Abstand zu einer Hornhautvorderseite liegt, und wobei die Krümmung der Hornhaut des Auges durch unterschiedliches Entfernen des Volumens an zumindest zwei Radien r1 und r2 um die optische Achse geändert wird.

Aus der DE 10 2006 053 120 A1 ist eine Behandlungsvorrichtung zur operativen Fehlsichtigkeitskorrektur eines Auges eines Patienten bekannt, die eine von einer Steuereinrichtung gesteuerte Lasereinrichtung aufweist, welche durch Einstrahlen von Laserstrahlung Hornhaut-Gewebe trennt, wobei die Steuereinrichtung die Lasereinrichtung zur Fokussierung der Laserstrahlung in die Hornhaut auf in einem Muster in der Hornhaut liegende Zielpunkte ansteuert und das Muster so wählt, dass damit ein Volumen in der Hornhaut isoliert ist, dessen Entfernung aus der Hornhaut die gewünschte Fehlsichtigkeitskorrektur bewirkt.

Der Erfindung liegt die Aufgabe zugrunde, Steuerdaten zum Steuern eines augenchirurgischen Lasers bereitzustellen, bei denen auf einfache Art und Weise ein Ausgleich einer Deformation eines Lentikels bereitgestellt wird, die durch ein Kontaktelement erzeugt wird.

Diese Aufgabe wird durch das erfindungsgemäße Verfahren, die erfindungsgemäßen Vorrichtungen, das erfindungsgemäße Computerprogramm sowie das erfindungsgemäße computerlesbare Medium gelöst. Vorteilhafte Ausgestaltungen mit zweckmäßigen Weiterbildungen der Erfindung sind in den jeweiligen Unteransprüchen angegeben, wobei vorteilhafte Ausgestaltungen des Verfahrens als vorteilhafte Ausgestaltungen der Behandlungsvorrichtung, der Steuereinrichtung, des Computerprogramms und des computerlesbaren Mediums und umgekehrt anzusehen sind.

Ein erster Aspekt der Erfindung betrifft ein Verfahren zum Bereitstellen von Steuerdaten eines augenchirurgischen Lasers einer Behandlungsvorrichtung für die Abtrennung eines Lentikels, wobei das Verfahren die folgenden, durch eine Steuereinrichtung durchgeführten Schritte aufweist. Unter einer Steuereinrichtung wird dabei ein Gerät, eine Gerätekomponente oder eine Gerätegruppe verstanden, das/die zum Empfangen und Auswerten von Signalen eingerichtet ist, sowie zum Bereitstellen, zum Beispiel Erzeugen, von Steuerdaten. Die Steuereinrichtung kann zum Beispiel als Steuerchip, Computerprogramm, Computerprogrammprodukt oder Steuergerät ausgestaltet sein. Durch die Steuereinrichtung erfolgt ein Ermitteln einer Lentikelgeometrie des abzutrennenden Lentikels, die eine Lentikelhöhe umfasst, aus vorbestimmten Fehlsichtigkeitsdaten eines menschlichen oder tierischen Auges, wobei die Lentikelgeometrie mittels eines zu korrigierenden Brechkraftwerts und eines Lentikeldurchmessers definiert wird, und ein Ermitteln eines Korrekturwertes zum Ausgleich einer Deformation des Lentikels, die durch mindestens ein Kontaktelement der Behandlungsvorrichtung erzeugt wird, wobei der Korrekturwert mittels zumindest einer vorangegangenen Messung der Behandlungsvorrichtung bestimmt wird. Ferner erfolgt durch die Steuereinrichtung ein Ermitteln einer Deformationsgeometrie des Lentikels, die eine Deformationshöhe umfasst, wobei die Deformationsgeometrie mittels des Brechkraftwerts und eines Deformationsdurchmessers definiert wird, wobei der Deformationsdurchmesser in Abhängigkeit des Lentikeldurchmessers und des Korrekturwertes berechnet wird. Durch die Steuereinrichtung erfolgt schließlich ein Bereitstellen von Steuerdaten zum Steuern des augenchirurgischen Lasers, wobei die Steuerdaten die Deformationsgeometrie zur Abtrennung des Lenktikels verwenden.

Mit anderen Worten wird zunächst aus zuvor bestimmten Fehlsichtigkeitsdaten die Lentikelgeometrie des abzutrennenden Lentikels bestimmt. Die Fehlsichtigkeitsdaten können zum Beispiel eine Ametropie beschreiben, also ein Abweichen der Brechkraft eines Auges beziehungsweise einer Hornhaut vom Idealwert, zum Beispiel eine Kurzsichtigkeit, Weitsichtigkeit oder Stabsichtigkeit. Die Fehlsichtigkeitsdaten können zum Beispiel einen Wert einer Brechkraft beschreiben, also eine Angabe in Dioptrien, oder zum Beispiel eine Abweichung einer Hornhautverkrümmung von einem Normalwert. Die Fehlsichtigkeitsdaten können zum Beispiel durch Abrufen der Fehlsichtigkeitsdaten von einem Datenspeicher oder Datenserver erfolgen, oder die Fehlsichtigkeitsdaten können zum Beispiel aus einem Messgerät, das Abmessungen der Hornhaut vornimmt und/oder die Ametropie feststellt, empfangen werden. Anhand der festgestellten Fehlsichtigkeitsdaten ermittelt die Steuereinrichtung eine Lentikelgeometrie, welche die Abmessungen eines Lentikels und dessen Position in der Kornea beschreibt. Das heißt, die Lentikelgeometrie, also die gewünschte Form des Lentikels und dessen Position in der Hornhaut, wird mittels der Fehlsichtigkeitsdaten bestimmt, wobei insbesondere eine Lentikelhöhe mittels eines zu korrigierenden Brechkraftwerts und eines Lentikeldurchmessers, der auch optische Zone genannt wird, definiert wird. Unter einem Lentikel wird dabei ein Volumenkörper der Kornea verstanden, also zum Beispiel eine diskusförmige Scheibe, die durch anteriore und posteriore Grenzflächen definiert sein kann. Die Grenzflächen können beispielsweise einen bikonvexen, bikonkaven, konkavkonvexen, konvexkonkaven, planparallelen, plankonvexen oder plankonkaven Volumenkörper ausbilden.

Nach oder vor diesem Verfahrensschritt kann die Steuereinrichtung einen Korrekturwert zum Ausgleich einer Deformation des Lentikels ermitteln, wobei die Deformation des Lentikels durch das Kontaktelement der Behandlungsvorrichtung erzeugt wird. Der Korrekturwert wird hierbei aus zumindest einer vorangegangenen Messung durch die Behandlungsvorrichtung bestimmt. Das heißt, der Korrekturwert wird empirisch bestimmt, aus einer zuvor durchgeführten Messung mit der Behandlungsvorrichtung.

Mittels des Korrekturwertes kann dann eine Deformationsgeometrie des Lentikels ermittelt werden, wobei hierfür aus dem Lentikeldurchmesser und dem Korrekturwert ein Deformationsdurchmesser bestimmt wird, mittels dem die zu korrigierende Lentikelhöhe erreicht wird. Aus dem Brechkraftwert und dem Deformationsdurchmesser kann dann die Deformationsgeometrie bestimmt werden, die insbesondere die Form des Lentikels beschreibt, die zum Ausgleich der Deformation durch das mindestens eine Kontaktelement der Behandlungsvorrichtung verwendet werden soll. Schließlich können die Steuerdaten zum Steuern des augenchirurgischen Lasers bereitgestellt werden, die statt der Lentikelgeometrie die Deformationsgeometrie zur Abtrennung des Lentikels verwenden.

Des Weiteren ist vorgesehen, dass der Korrekturwert aus einer Abweichung der Lentikelhöhe, die gemäß der Lentikelgeometrie erwartet wird, und einer erreichten Lentikelhöhe ermittelt wird, wobei die erreichte Lentikelhöhe nach einer Anwendung der Behandlungsvorrichtung ohne Verwendung der Deformationsgeometrie bestimmt wird. Mit anderen Worten kann die Abweichung bestimmt werden, die zwischen der erwarteten Lentikelhöhe, das heißt der geplanten Lentikelhöhe, und der erreichten Lentikelhöhe vorliegt, wenn die Deformationsgeometrie nicht verwendet wird.

Hieraus kann dann der Korrekturwert bestimmt werden. Beispielsweise kann aus der Lentikelgeometrie geplant gewesen sein, dass eine Lentikelhöhe von 100 Mikrometer bei der Behandlung abgetrennt wird, wobei hier mit Lentikelhöhe die maximale Höhe des Lentikels gemeint ist. Aufgrund der Deformation des Lentikels kann jedoch beispielsweise eine Lentikelhöhe von 107 Mikrometer abgetrennt werden, wobei der Brechkraftwert zuvor manuell angepasst worden sein kann. Das würde eine Abweichung von 7 Prozent ergeben, wobei der Korrekturwert vorzugsweise so ermittelt wird, dass diese 7 Prozent Abweichung ausgeglichen werden. Um diese Abweichung zwischen den Lentikelhöhen zu bestimmen, kann die Messung ohne Verwendung der Deformationsgeometrie vorzugsweise an künstlichen Hornhäuten und/oder an tierischen Hornhäuten und/oder an humanen Spenderhornhäuten durchgeführt werden. Hierdurch ergibt sich der Vorteil, dass der Korrekturwert mittels einer einfachen Messung bestimmt werden kann.

Durch diesen Aspekt der Erfindung ergibt sich der Vorteil, dass Behandlungsergebnisse weiter verbessert werden können, insbesondere eine abzutrennende Höhe des Lentikels richtig bestimmt werden kann, um Deformationseffekte aufgrund eines Kontaktelements auszugleichen, wobei hierfür beispielsweise zuvor eine manuelle Anpassung des zu korrigierenden Brechkraftwerts durchgeführt sein kann. Das heißt, dass beispielsweise ein Arzt den Brechkraftwert vor der Behandlung aufgrund seiner Erfahrung zum Ausgleich der Deformation durch das Kontaktelement wie gewohnt anpassen kann und durch das erfindungsgemäße Verfahren zusätzlich eine darauf abgestimmte Deformationsgeometrie des Lentikels bestimmt wird, durch die eine aus der Hornhaut zu entnehmende Gewebemenge optimiert wird.

Vorteilhaft ist es, wenn der Korrekturwert aus mehreren vorangegangenen Messungen der Lentikelhöhe und der erreichten Lentikelhöhe statistisch bestimmt wird, insbesondere mittels einer linearen Regression. Das hat den Vorteil, dass der Korrekturwert robuster gegenüber statistischen Abweichungen wird. Beispielsweise können mehrere Messungen durchgeführt werden, zum Beispiel für mehrere geplante Lentikelhöhen zwischen 40 und 120 Mikrometer, die jeweils mit den erreichten Lentikelhöhen verglichen werden. Hierzu können bekannte statistische Verfahren angewandt werden, insbesondere eine lineare Regression, deren Steigung die Abweichung zwischen den Lentikelhöhen darstellen kann.

Weiterhin vorteilhaft ist, wenn die erreichte Lentikelhöhe zu mindestens zwei unterschiedlichen Zeitpunkten bestimmt wird, wobei der Korrekturwert aus einem Mittelwert der Abweichungen zu den mindestens zwei unterschiedlichen Zeitpunkten ermittelt wird. Insbesondere kann einer der Zeitpunkte kurz nach der Behandlung sein, beispielsweise einen Tag oder eine Woche nach der Behandlung, und der andere Zeitpunkt kann später, beispielsweise nach einem Monat, sein. Die erreichte Lentikelhöhe zu diesen zwei oder auch mehreren Zeitpunkten kann dann gemittelt werden, um die Abweichung von der zu erwartenden Lentikelhöhe zu bestimmen, woraus dann der Korrekturwert bestimmt werden kann. Das hat den Vorteil, dass der Korrekturwert durch die Verwendung mehrerer Messungen statistisch robuster wird.

Gemäß einer weiteren vorteilhaften Ausgestaltungsform ist vorgesehen, dass der Korrekturwert mit einem Brechkraftkorrekturwert kombiniert wird, wobei der Brechkraftkorrekturwert aus einer Abweichung des zu korrigierenden Brechkraftwerts und eines erreichten Brechkraftwerts ermittelt wird, insbesondere mittels einer linearen Regression aus mehreren vorangegangenen Messungen und einem Mittelwert aus Brechkraftwertabweichungen zu unterschiedlichen Zeitpunkten, wobei der erreichte Brechkraftwert nach einer Anwendung der Behandlungsvorrichtung bestimmt wird. Mit anderen Worten kann der Korrekturwert, der aus einer Abweichung der erwarteten und erreichten Lentikelhöhe ermittelt wird, mit einem Brechkraftkorrekturwert kombiniert werden, wobei der Brechkraftkorrekturwert aus einer Abweichung des geplanten und erreichten Brechkraftwerts ermittelt wird. Der Brechkraftkorrekturwert kann dabei vorzugsweise mittels der gleichen statistischen Methoden bestimmt werden, wie der Korrekturwert. Durch diese Ausgestaltungsform ergibt sich der Vorteil, dass bei der Ermittlung der Deformationsgeometrie zusätzlich eine Abweichung des Brechkraftwerts berücksichtigt werden kann. Aufgrund der Deformation des Lentikels durch das Kontaktelement ist beispielsweise vorgesehen, dass der Arzt den Brechkraftwert aus Erfahrungswerten entsprechend anpasst, um einen Ausgleich zu der Deformation zu erreichen. Diese Anpassung des Brechkraftwertes kann jedoch nicht optimal sein und es kann immer noch eine Abweichung vom zu korrigierenden Brechkraftwert zu dem erreichten Brechkraftwert vorliegen, die aus Messungen mit der Behandlungsvorrichtung bestimmt werden kann. Um diese Abweichung der Brechkraftwerte zusätzlich auszugleichen, kann daher der Brechkraftkorrekturwert mit den Korrekturwert kombiniert werden, indem der Korrekturwert beispielsweise mit dem Kehrwert des Brechkraftkorrekturwertes multipliziert wird. Beispielsweise können die Brechkraftwerte im Mittel eine 7 Prozentige Abweichung voneinander aufweisen, wobei diese 7 Prozent dann der Brechkraftkorrekturwert sein können, der mit dem Korrekturwert kombiniert wird.

Vorzugsweise liegt der Korrekturwert in einem Bereich von 0,7 bis 1,5, insbesondere in einem Bereich von 1,0 bis 1,4. Das heißt, wenn zwischen der Lentikelhöhe, die aus der Lentikelgeometrie erwartet wird, und der ermittelten Lentikelhöhe eine Abweichung von 10 Prozent festgestellt wurde, kann der Korrekturwert bei 1,1 liegen. Zusammen mit dem oben genannten Brechkraftkorrekturwert würde das einen Gesamtkorrekturwert von etwa 1,18 ergeben.

Gemäß einer weiteren vorteilhaften Ausgestaltungsform wird der Deformationsdurchmesser mittels einer Multiplikation des Lentikeldurchmessers mit einem vom Korrekturwert abhängigen Parameter berechnet, wobei der Parameter mittels eines theoretischen Lentikelgeometriemodells, insbesondere mittels der Munnerlyn Formel, bestimmt wird. Mit anderen Worten kann zur Berechnung der Deformationsgeometrie der Deformationsdurchmesser aus dem Lentikeldurchmesser, das heißt dem geplanten Lentikeldurchmesser, berechnet werden, indem der Lentikeldurchmesser mit einem Parameter multipliziert wird, wobei der Parameter vom Korrekturwert abhängig ist. Mit dem vom Korrekturwert abhängigen Parameter ist gemeint, dass dieser eine mathematische Rechenoperation mit dem Korrekturwert umfasst, insbesondere eine Potenz des Korrekturwerts. Insbesondere kann der vom Korrekturwert abhängige Parameter aus einem Lentikelgeometriemodell bestimmt werden, vorzugsweise aus dem Lentikelgeometriemodell durch das die Lentikelgeometrie ermittelt wird, beispielsweise mittels der Munnerlyn Formel.

Gemäß einer weiteren vorteilhaften Ausgestaltungsform wird für die Deformationshöhe der Deformationsgeometrie eine Höhe ermittelt, die einer Multiplikation der Lentikelhöhe mit dem Kehrwert des Korrekturwerts entspricht, indem der Deformationsdurchmesser mittels einer Division des Lentikeldurchmessers durch die Quadratwurzel des Korrekturwertes berechnet wird.

Mit anderen Worten reduziert sich die Deformationshöhe auf eine Höhe, die der Lentikelhöhe geteilt durch den Korrekturwert entspricht. Dafür wird der Deformationsdurchmesser mittels einer Division des Lentikeldurchmessers durch den "Korrekturwert hoch ½" berechnet. Die Quadratwurzel des Korrekturwertes wird dabei insbesondere aus einer analytischen Berechnung des Lentikelgeometriemodells erhalten, vorzugsweise aus einer Berechnung mittels der Munnerlyn Formel.

Ein zweiter Aspekt der vorliegenden Erfindung betrifft eine Steuereinrichtung, die dazu eingerichtet ist, eines der oben beschriebenen Verfahren durchzuführen. Es ergeben sich die oben aufgeführten Vorteile. Die Steuereinrichtung kann zum Beispiel als Steuerchip, Steuergerät oder Anwenderprogramm ("App") ausgestaltet sein. Die Steuereinrichtung kann vorzugsweise eine Prozessoreinrichtung aufweisen und/oder einen Datenspeicher. Unter einer Prozessoreinrichtung wird ein Gerät oder eine Gerätekomponente zur elektronischen Datenverarbeitung verstanden. Die Prozessoreinrichtung kann zum Beispiel mindestens einen Mikrocontroller und/oder mindestens einen Mikroprozessor aufweisen. Auf dem optionalen Datenspeicher kann vorzugsweise ein Programmcode zum Durchführen des erfindungsgemäßen Verfahrens abgelegt sein. Der Programmcode kann dann dazu ausgelegt sein, bei Ausführung durch die Prozessoreinrichtung die Steuereinrichtung dazu zu veranlassen, eine der oben beschriebenen Ausführungsformen eines oder beider erfindungsgemäßer Verfahren durchzuführen.

Ein dritter Aspekt der vorliegenden Erfindung betrifft eine Behandlungsvorrichtung mit mindestens einem augenchirurgischen Laser für die Abtrennung eines vordefinierten Hornhautvolumens mit vordefinierten Grenzflächen eines menschlichen oder tierischen Auges mittels Photodisruption, und mindestens einer Steuereinrichtung für den oder die Laser, die ausgebildet ist, die Schritte des Verfahrens gemäß dem ersten Aspekt der Erfindung auszuführen. Die erfindungsgemäße Behandlungsvorrichtung ermöglicht es, dass die bei der Verwendung üblicher ablativer Behandlungsvorrichtungen auftretenden Nachteile zuverlässig reduziert oder sogar vermieden werden.

In einer weiteren vorteilhaften Ausgestaltung der erfindungsgemäßen Behandlungsvorrichtung kann der Laser dazu geeignet sein, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 Kilohertz (KHz), vorzugsweise zwischen 100 KHz und 100 Megahertz (MHz), abzugeben. Ein solcher Femtosekundenlaser ist zur Herstellung von Volumenkörpern innerhalb der Kornea besonders gut geeignet. Die Verwendung von photodisruptiven Lasern bei dem erfindungsgemäßen Verfahren weist zudem den Vorteil auf, dass die Bestrahlung der Kornea nicht in einem Wellenlängenbereich unter 300 nm erfolgen muss. Dieser Bereich wird in der Lasertechnik unter dem Begriff "tiefes Ultraviolett" subsumiert. Dadurch wird vorteilhafterweise vermieden, dass durch diese sehr kurzwelligen und energiereichen Strahlen eine unbeabsichtigte Schädigung der Kornea erfolgt. Photodisruptive Laser der hier verwendeten Art bringen üblicherweise gepulste Laserstrahlung mit einer Pulsdauer zwischen 1 fs und 1 ns in das Korneagewebe ein. Dadurch kann die für den optischen Durchbruch notwendige Leistungsdichte des jeweiligen Laserpulses räumlich eng begrenzt werden, so dass eine hohe Schnittgenauigkeit bei der Erzeugung der Grenzflächen ermöglicht wird. Als Wellenlängenbereich kann insbesondere auch der Bereich zwischen 700 nm und 780 nm gewählt werden.

In weiteren vorteilhaften Ausgestaltungen der erfindungsgemäßen Behandlungsvorrichtung kann die Steuereinrichtung mindestens eine Speichereinrichtung zur zumindest temporären Speicherung von mindestens einem Steuerdatensatz aufweisen, wobei der oder die Steuerdatensätze Steuerdaten zur Positionierung und/oder zur Fokussierung einzelner Laserpulse in der Hornhaut/Kornea umfassen; und kann mindestens eine Strahleinrichtung zur Strahlführung und/oder Strahlformung und/oder Strahlablenkung und/oder Strahlfokussierung eines Laserstrahls des Lasers aufweisen. Die genannten Steuerdatensätze werden dabei üblicherweise anhand einer gemessenen Topografie und/oder Pachymetrie und/oder Morphologie der zu behandelnden Kornea und der Art der zu korrigierenden Fehlsichtigkeit, erzeugt.

Weitere Merkmale und deren Vorteile sind den Beschreibungen des ersten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Ein vierter Aspekt der Erfindung betrifft ein Computerprogramm, umfassend Befehle, die bewirken, dass die Behandlungsvorrichtung gemäß dem dritten Erfindungsaspekt die Verfahrensschritte gemäß dem ersten Erfindungsaspekt und/oder die Verfahrensschritte gemäß dem zweiten Erfindungsaspekt ausführt.

Ein fünfter Aspekt der Erfindung betrifft ein computerlesbares Medium, auf dem das Computerprogramm gemäß dem vierten Erfindungsaspekt gespeichert ist. Weitere Merkmale und deren Vorteile sind den Beschreibungen des ersten bis vierten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Die Erfindung wird durch die Ansprüche definiert.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Darstellung einer erfindungsgemäßen Behandlungsvorrichtung gemäß einer beispielhaften Ausführungsform;
- Fig. 2: ein beispielhaftes Streudiagramm von erwarteten und erreichten Lentikelhöhen;
- Fig. 3: ein schematisches Verfahrensdiagramm gemäß einer beispielhaften Ausführungsform.

In den Figuren sind gleiche oder funktionsgleiche Elemente mit den gleichen Bezugszeichen versehen.

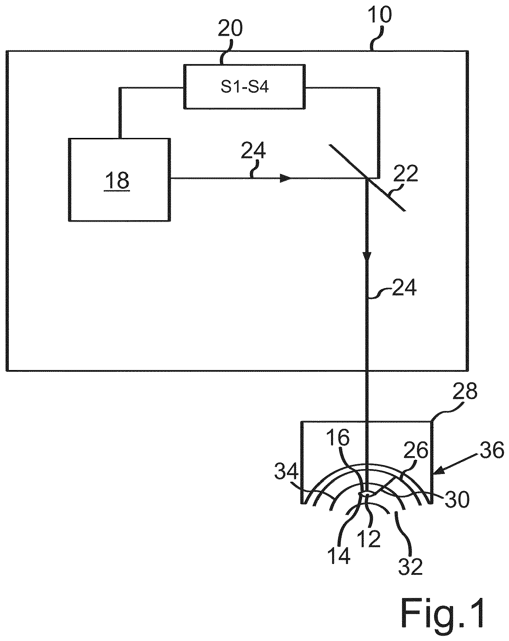

Die Figur 1 zeigt eine schematische Darstellung einer Behandlungsvorrichtung 10 mit einem augenchirurgischen Laser 18 für die Abtrennung eines durch eine Lentikelgeometrie vordefinierten Hornhautvolumens/Korneavolumens einer Hornhaut (Kornea) eines menschlichen oder tierischen Auges 36, also eines Lentikels 12, der auch als Volumenkörper bezeichnet werden kann, mit vordefinierten Grenzflächen 14, 16 mittels Photodisruption. Man erkennt, dass neben dem Laser 18 eine Steuereinrichtung 20 für den Laser 18 ausgebildet sein kann, sodass dieser gepulste Laserpulse beispielsweise in einem vordefinierten Muster in die Kornea des Auges 36 abgibt, wobei die ermittelten Grenzflächen 14, 16 des zu bildenden Lentikels 12 durch zum Beispiel ein vordefiniertes Muster mittels Photodisruption erzeugt werden können. Alternativ kann die Steuereinrichtung 20 eine in Bezug auf die Behandlungsvorrichtung 10 externe Steuereinrichtung 20 sein.

Die ermittelten Grenzflächen 14, 16 bilden in dem dargestellten Ausführungsbeispiel einen Lentikel 12 aus, wobei die Position des Lentikels 12 in diesem Ausführungsbeispiel derart gewählt ist, dass dieser zum Beispiel innerhalb einer Stroma 32 der Kornea liegen kann. Des Weiteren ist aus Figur 1 erkennbar, dass zwischen der Stroma 32 und einem Epithelium die so genannte Bowman-Membran 34 ausgebildet sein kann.

Des Weiteren zeigt die Figur 1, dass der durch den Laser 18 erzeugte Laserstrahl 24 mittels einer Strahleinrichtung 22, nämlich einer Strahlablenkungsvorrichtung, wie zum Beispiel einem Rotationscanner, in Richtung einer Oberfläche 26 der Hornhaut abgelenkt wird. Die Strahlablenkvorrichtung wird ebenfalls durch die Steuereinrichtung 20 gesteuert, um die ermittelten Grenzflächen 14, 16, vorzugsweise auch Inzisionen oder Schnitte 30, entlang von vorgegebenen Inzisionsverläufen zu erzeugen.

Bei dem dargestellten Laser 18 kann es sich vorzugsweise um einen photodisruptiven Laser handeln, der ausgebildet ist, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben. Die Steuereinrichtung 20 weist optional zudem eine Speichereinrichtung (nicht dargestellt) zur zumindest temporären Speicherung von mindestens einem Steuerdatensatz auf, wobei der oder die Steuerdatensätze Steuerdaten zur Positionierung und/oder zur Fokussierung einzelner Laserpulse in der Kornea umfassen. Die Positionsdaten und/oder Fokussierungsdaten der einzelnen Laserpulse, das heißt, die Lentikelgeometrie des abzutrennenden Lentikels 12, wird anhand vorbestimmter Fehlsichtigkeitsdaten erzeugt, insbesondere aus einer zuvor gemessenen Topografie und/oder Pachymetrie und/oder der Morphologie der Hornhaut oder der zu erzeugenden optischen Fehlsichtigkeitskorrektur beispielhaft innerhalb der Stroma 32 des Auges 36 erzeugt. Zum Feststellen der Fehlsichtigkeitsdaten, die zum Beispiel einen Wert in Dioptrien angeben können oder andere, geeignete Daten zum Beschreiben der Fehlsichtigkeit, kann die Steuereinrichtung 20 zum Beispiel die entsprechenden Daten von einem Datenserver empfangen, oder die Fehlsichtigkeitsdaten können als Dateneingabe festgestellt werden. Die Lentikelgeometrie des abzutrennenden Lentikels 12 zur Korrektur der Fehlsichtigkeit umfasst eine Lentikelhöhe und wird insbesondere mittels eines zu korrigierenden Brechkraftwerts, der auch als geplanter Brechkraftwert bezeichnet werden kann, und eines Lentikeldurchmessers, der auch als optische Zone bezeichnet wird, definiert. Die Lentikelhöhe oder auch Lentikeldicke ist ein variabler Wert, der die Höhe des Lentikels in einer Anterior-Posterior-Richtung, das heißt parallel zu einer optischen Achse des Auges 36, in Abhängigkeit einer Position senkrecht zur optischen Achse angibt, insbesondere in Abhängigkeit einer Position in einer Radialrichtung des Lentikels. Ein genauer Verlauf der Lentikelhöhe in Abhängigkeit der Position in Radialrichtung kann beispielsweise mittels eines theoretischen Lentikelgeometriemodells, insbesondere mittels der Munnerlyn Formel, bestimmt werden.

Ferner kann ein Kontaktelement 28 bereitgestellt sein, das zu der Behandlungsvorrichtung 10 gehören kann. Alternativ kann das Kontaktelement 28 auch separat zu der Behandlungsvorrichtung 10 vorgesehen sein. Das Kontaktelement 28, das auch als Patienteninterface oder Fixiersystem bezeichnet werden kann, dient dazu, das Auge 36 für die Behandlung zu fixieren. Hierzu kann das Kontaktelement 28 eine plankonkave Linse aufweisen, die auf das Auge 36 zur Fixierung angepasst wird. Durch die Fixierung mittels des Kontaktelements 28 kann es jedoch dazu kommen, dass sich die Kornea verformt und somit die ermittelte Lentikelgeometrie zum Abtrennen des Lentikels 12 nicht mehr optimal auf die vorbestimmten Fehlsichtigkeitsdaten zutrifft. Daher kann es vorkommen, dass eine geplante beziehungsweise zu korrigierende Lentikelhöhe von einer erreichten Lentikelhöhe nach der Behandlung mit der Behandlungsvorrichtung 10 abweicht.

Einem Benutzer der Behandlungsvorrichtung 10, insbesondere einem Arzt, kann eine solche Abweichung, die durch das Kontaktelement 28 entsteht, bekannt sein. Daher wird der Arzt vorzugsweise den zu korrigierenden Brechkraftwert bei der Planung nach seinen Erfahrungswerten derart anpassen, zum Beispiel durch Erhöhen des Brechkraftwerts, dass nach der Behandlung mit der Behandlungsvorrichtung der erreichte Brechkraftwert der gewünschten Korrektur entspricht.

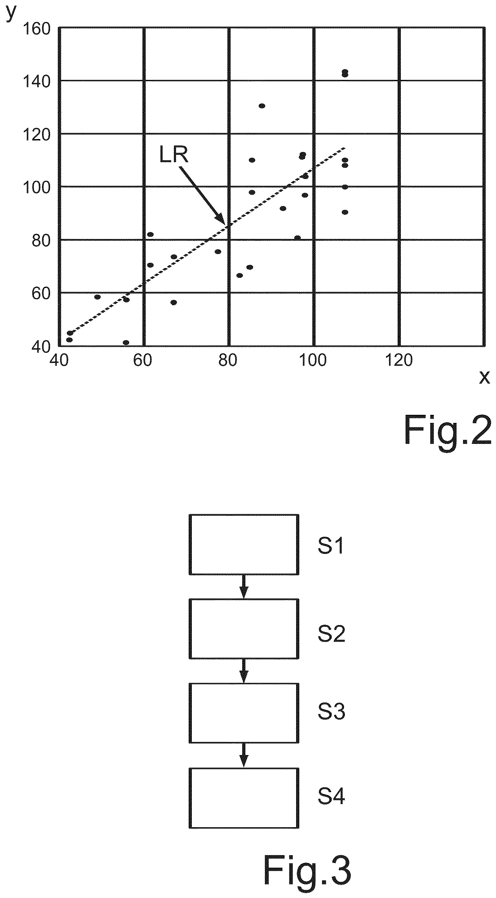

Die Deformation durch das Kontaktelement 28 kann dazu führen, dass die entfernte Lentikelhöhe nicht optimal ist. Beispielsweise kann eine erreichte Lentikelhöhe größer sein als erforderlich oder geplant. Das heißt es wäre mehr Gewebe aus der Hornhaut entnommen worden als erforderlich. Dies ist beispielsweise in dem Streudiagramm der Figur 2 dargestellt. In Figur 2 ist auf der x-Achse die Lentikelhöhe (in Mikrometer) aufgetragen, die durch die Behandlung erwartet wird und auf der y-Achse die erreichte Lentikelhöhe (in Mikrometer) einen Tag nach der Behandlung. Eine lineare Regressionsgerade LR kann in diesem Beispiel eine Steigung von 1,07 aufweisen.

Um diese Abweichung auszugleichen, kann vorgesehen sein, dass die Steuereinrichtung 20 die nachfolgend beschriebenen Verfahrensschritte S1 bis S4 durchführt, die beispielsweise in Figur 3 als Verfahrensdiagramm dargestellt sind. In dem Schritt S1 wird, wie oben beschrieben, die Lentikelgeometrie des abzutrennenden Lentikels 12 aus den vorbestimmten Fehlsichtigkeitsdaten bestimmt, wobei die Lentikelgeometrie mittels des zu korrigierenden Brechkraftwerts und des Lentikeldurchmessers definiert wird. Der zu korrigierende Brechkraftwert kann dabei nach Erfahrungswerten vom Arzt angepasst sein. Anschließend kann in dem Schritt S2 ein Korrekturwert zum Ausgleich der Deformation durch das Kontaktelement 28 aus zumindest einer vorangegangenen Messung mit der Behandlungsvorrichtung 10 bestimmt werden. Vorzugsweise kann der Korrekturwert statistisch aus den Messungen der Figur 2 bestimmt werden, indem die Steigung der Regressionsgeraden LR als Korrekturwert verwendet wird, also im Beispiel von oben 1,07.

Die zuvor gezeigten Zahlen sollen jedoch nur Beispiele des Korrekturwertes darstellen und der Korrekturwert kann beispielsweise je nach Behandlungsvorrichtung und dazugehörigem Kontaktelement unterschiedlich sein. Auch eine Art der Behandlung, das heißt in Abhängigkeit der Fehlsichtigkeitsdaten, und eine Anpassung des Brechkraftwerts durch den Arzt können den Korrekturwert verändern. Insbesondere kann der Korrekturwert in einem Bereich von 0,7 bis 1,5 liegen, vorzugsweise in einem Bereich von 1,0 bis 1,4.

Nach Ermittlung des Korrekturwerts kann durch die Steuereinrichtung in dem Schritt S3 eine Deformationsgeometrie des Lentikels 12 ermittelt werden, wobei für die Deformationsgeometrie der Brechkraftwert der Lentikelgeometrie, insbesondere der Brechkraftwert, der durch den Arzt festgesetzt wurde, übernommen werden kann. Als ein Deformationsdurchmesser der Deformationsgeometrie kann der Lentikeldurchmessers durch einem vom Korrekturwert abhängigen Parameter geteilt werden, wobei der Parameter mittels eines theoretischen Lentikelgeometriemodells bestimmt wird. Beispielsweise kann das Lentikelgeometriemodell dasjenige Modell sein, mittels dem zuvor die Lentikelgeometrie bestimmt wurde. Vorzugsweise kann das Lentikelgeometriemodell die Munnerlyn Formel sein, wobei der vom Korrekturwert abhängige Parameter dabei die Quadratwurzel des Korrekturwertes ist. Das heißt, dass der Deformationsdurchmesser berechnet wird, indem der Lentikeldurchmesser mit der Quadratwurzel des Korrekturwertes, also im Beispiel oben mit der Wurzel von 1,07 geteilt wird. Folglich wird mittels der Deformationsgeometrie eine Deformationshöhe ermittelt, die der Lentikelhöhe geteilt durch den Korrekturwert entspricht.

Die so bestimmte Deformationsgeometrie kann dann in einem Schritt S4 in Form von Steuerdaten zum Steuern des augenchirurgischen Lasers 18 von der Steuereinrichtung 20 bereitgestellt werden. Mittels der so bereitgestellten Steuerdaten, die die Deformationsgeometrie zur Abtrennung des Lentikels 12 verwenden, kann auf einfache Art und Weise ein Ausgleich der Deformation durch das Kontaktelement 28 erreicht werden, wobei eine Höhe des abzutrennenden Lentikels 12 klein gehalten werden kann. Hierdurch kann eine Behandlung mit der Behandlungsvorrichtung 10 verbessert werden.

## Patentansprüche

1. Verfahren zum Bereitstellen von Steuerdaten eines augenchirurgischen Lasers (18) einer Behandlungsvorrichtung (10) für die Abtrennung eines Lentikels (12), wobei das Verfahren die folgenden, durch eine Steuereinrichtung durchgeführten Schritte aufweist:
- Ermitteln (S1) einer Lentikelgeometrie des abzutrennenden Lentikels (12), die eine Lentikelhöhe umfasst, aus vorbestimmten Fehlsichtigkeitsdaten eines menschlichen oder tierischen Auges (36), wobei die Lentikelgeometrie mittels eines zu korrigierenden Brechkraftwerts und eines Lentikeldurchmessers definiert wird;
- Ermitteln (S2) eines Korrekturwertes zum Ausgleich einer Deformation des Lentikels (12), die durch mindestens ein Kontaktelement (28) der Behandlungsvorrichtung (10) erzeugt wird, wobei der Korrekturwert mittels zumindest einer vorangegangenen Messung der Behandlungsvorrichtung (10) bestimmt wird;
- Ermitteln (S3) einer Deformationsgeometrie des Lentikels (12), die eine Deformationshöhe umfasst, wobei die Deformationsgeometrie mittels des Brechkraftwerts und eines Deformationsdurchmessers definiert wird, wobei der Deformationsdurchmesser in Abhängigkeit des Lentikeldurchmessers und des Korrekturwerts berechnet wird;
- Bereitstellen (S4) von Steuerdaten zum Steuern des augenchirurgischen Lasers (18), die die Deformationsgeometrie zur Abtrennung des Lentikels (12) verwenden **gekennzeichnet dadurch, dass** der Korrekturwert aus einer Abweichung der Lentikelhöhe, die gemäß der Lentikelgeometrie erwartet wird, und einer erreichten Lentikelhöhe ermittelt wird, wobei die erreichte Lentikelhöhe nach einer Anwendung der Behandlungsvorrichtung (10) ohne Verwendung der Deformationsgeometrie bestimmt wird.

2. Verfahren nach Anspruch 1
**dadurch gekennzeichnet, dass**
der Korrekturwert aus mehreren vorangegangenen Messungen der Lentikelhöhe und der erreichten Lentikelhöhe statistisch bestimmt wird, insbesondere mittels einer linearen Regression (LR).

3. Verfahren nach einem der vorhergehenden Ansprüche
**dadurch gekennzeichnet, dass**
die erreichte Lentikelhöhe zu mindestens zwei unterschiedlichen Zeitpunkten bestimmt wird, wobei der Korrekturwert aus einem Mittelwert der Abweichungen zu den mindestens zwei unterschiedlichen Zeitpunkten ermittelt wird.

4. Verfahren nach einem der vorhergehenden Ansprüche
**dadurch gekennzeichnet, dass**
der Korrekturwert mit einem Brechkraftkorrekturwert kombiniert wird, wobei der Brechkraftkorrekturwert aus einer Abweichung des zu korrigierenden Brechkraftwerts und eines erreichten Brechkraftwerts ermittelt wird, insbesondere mittels einer linearen Regression aus mehreren vorangegangenen Messungen und einem Mittelwert aus Brechkraftwertabweichungen zu unterschiedlichen Zeitpunkten, wobei der erreichte Brechkraftwert nach einer Anwendung der Behandlungsvorrichtung (10) bestimmt wird.

5. Verfahren nach einem der vorhergehenden Ansprüche,
**dadurch gekennzeichnet, dass**
der Korrekturwert in einem Bereich von 0,7 bis 1,5 liegt, insbesondere in einem Bereich von 1,0 bis 1,4.

6. Verfahren nach einem der vorhergehenden Ansprüche,
**dadurch gekennzeichnet, dass**
der Deformationsdurchmesser mittels einer Multiplikation des Lentikeldurchmessers mit einem vom Korrekturwert abhängigen Parameter berechnet wird, wobei der Parameter mittels eines theoretischen Lentikelgeometriemodells, insbesondere mittels der Munnerlyn Formel, bestimmt wird.

7. Verfahren nach Anspruch 6,
**dadurch gekennzeichnet, dass**
für die Deformationshöhe der Deformationsgeometrie eine Höhe ermittelt wird, die einer Multiplikation der Lentikelhöhe mit dem Kehrwert des Korrekturwerts entspricht, indem der Deformationsdurchmesser mittels einer Division des Lentikeldurchmessers durch die Quadratwurzel des Korrekturwertes berechnet wird.

8. Steuereinrichtung (20), die dazu ausgebildet ist, ein Verfahren nach einem der vorhergehenden Ansprüche durchzuführen.

9. Behandlungsvorrichtung (10) mit mindestens einem augenchirurgischen Laser (18) für die Abtrennung eines Lentikels (12) eines menschlichen oder tierischen Auges (36) mittels Photodisruption und mindestens einer Steuereinrichtung (20) nach Anspruch 8.

10. Behandlungsvorrichtung (10) nach Anspruch 9,
**dadurch gekennzeichnet, dass**
der Laser (18) dazu ausgebildet ist, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

11. Behandlungsvorrichtung (10) nach einem der Ansprüche 9 oder 10,
**dadurch gekennzeichnet, dass** die Steuereinrichtung (20)
- mindestens eine Speichereinrichtung zur zumindest temporären Speicherung von mindestens einem Steuerdatensatz aufweist, wobei der oder die Steuerdatensätze Steuerdaten zur Positionierung und/oder Fokussierung einzelner Laserpulse in der Kornea umfassen; und
- mindestens eine Strahleinrichtung (22) zur Strahlführung und/oder Strahlformung und/oder Strahlablenkung und/oder Strahlfokussierung eines Laserstrahls (24) des Lasers (18) umfasst.

12. Computerprogramm, umfassend Befehle, die bewirken, dass die Behandlungsvorrichtung (10) gemäß einem der Ansprüche 9 bis 11 ein Verfahren nach einem der Ansprüche 1 bis 7 ausführt.

13. Computerlesbares Medium, auf welchem das Computerprogramm nach Anspruch 12 gespeichert ist.

## Claims

1. A method for providing control data of an eye surgical laser (18) of a treatment apparatus (10) for the separation of a lenticule (12), wherein the method comprises the following steps performed by a control device:
- ascertaining (S1) a lenticule geometry of the lenticule (12) to be separated, which includes a lenticule height, from predetermined visual disorder data of a human or animal eye (36), wherein the lenticule geometry is defined by means of a refractive power value to be corrected and a lenticule diameter;
- ascertaining (S2) a correction value for compensating for a deformation of the lenticule (12), which is generated by at least one contact element (28) of the treatment apparatus (10), wherein the correction value is determined by means of at least one preceding measurement of the treatment apparatus (10);
- ascertaining (S3) a deformation geometry of the lenticule (12), which includes a deformation height, wherein the deformation geometry is defined by means of the refractive power value and a deformation diameter, wherein the deformation diameter is calculated depending on the lenticule diameter and the correction value;
- providing (S4) control data for controlling the eye surgical laser (18), which uses the deformation geometry for separating the lenticule (12),
**characterized in that**
the correction value is ascertained from a deviation of the lenticule height, which is expected according to the lenticule geometry, and an achieved lenticule height, wherein the achieved lenticule height is determined after an application of the treatment apparatus (10) without use of the deformation geometry.

2. The method according to claim 1,
**characterized in that**
the correction value is statistically determined from multiple preceding measurements of the lenticule height and the achieved lenticule height, in particular by means of a linear regression (LR).

3. The method according to any one of the preceding claims,
**characterized in that**
the achieved lenticule height is determined at least at two different points of time, wherein the correction value is ascertained from an average value of the deviations at the at least two different points of time.

4. The method according to any one of the preceding claims,
**characterized in that**
the correction value is combined with a refractive power correction value, wherein the refractive power correction value is ascertained from a deviation of the refractive power value to be corrected and an achieved refractive power value, in particular by means of a linear regression from multiple preceding measurements and an average value from refractive power value deviations at different points of time, wherein the achieved refractive power value is determined after an application of the treatment apparatus (10).

5. The method according to any one of the preceding claims,
**characterized in that**
the correction value is in a range from 0.7 to 1.5, in particular in a range from 1.0 to 1.4.

6. The method according to any one of the preceding claims,
**characterized in that**
the deformation diameter is calculated by means of a multiplication of the lenticule diameter by a parameter depending on the correction value, wherein the parameter is determined by means of a theoretical lenticule geometry model, in particular by means of the Munnerlyn formula.

7. The method according to claim 6,
**characterized in that**
a height is ascertained for the deformation height of the deformation geometry, which corresponds to a multiplication of the lenticule height by the reciprocal value of the correction value, **in that** the deformation diameter is calculated by means of a division of the lenticule diameter by the square root of the correction value.

8. A control device (20), which is formed to perform a method according to any one of the preceding claims.

9. A treatment apparatus (10) with at least one eye surgical laser (18) for the separation of a lenticule (12) of a human or animal eye (36) by means of photodisruption and at least one control device (20) according to claim 8.

10. The treatment apparatus (10) according to claim 9,
**characterized in that**
the laser (18) is formed to emit laser pulses in a wavelength range between 300 nm and 1400 nm, preferably between 700 nm and 1200 nm, at a respective pulse duration between 1 fs and 1 ns, preferably between 10 fs and 10 ps, and a repetition frequency of greater than 10 kHz, preferably between 100 kHz and 100 MHz.

11. The treatment apparatus (10) according to any one of claims 9 or 10, **characterized in that** the control device (20)
- comprises at least one storage device for at least temporary storage of at least one control dataset, wherein the control dataset or datasets include control data for positioning and/or focusing individual laser pulses in the cornea; and
- includes at least one beam device (22) for beam guidance and/or beam shaping and/or beam deflection and/or beam focusing of a laser beam (24) of the laser (18).

12. A computer program including instructions, which cause the treatment apparatus (10) according to any one of claims 9 to 11 to execute a method according to any one of claims 1 to 7.

13. A computer-readable medium, on which the computer program according to claim 12 is stored.

## Revendications

1. Procédé pour fournir des données de commande d'un laser chirurgical ophtalmique (18) d'un dispositif de traitement (10) pour la séparation d'un lenticule (12), le procédé comportant les étapes suivantes réalisées par un dispositif de commande :
déterminer (S1) une géométrie de lenticule du lenticule (12) à séparer, laquelle géométrie comprenant une hauteur de lenticule, à partir de données prédéterminées concernant un trouble visuel d'un oeil humain ou animal (36), la géométrie de lenticule étant définie au moyen d'une valeur de réfringence à corriger et d'un diamètre de lenticule ;
déterminer (S2) une valeur de correction pour compenser une déformation du lenticule (12) qui est générée par au moins un élément de contact (28) du dispositif de traitement (10), la valeur de correction étant établie au moyen d'au moins une mesure précédente du dispositif de traitement (10) ;
déterminer (S3) une géométrie de déformation du lenticule (12) comprenant une hauteur de déformation, la géométrie de déformation étant définie au moyen de la valeur de réfringence et d'un diamètre de déformation, le diamètre de déformation étant calculé en fonction du diamètre de lenticule et de la valeur de correction ;
fournir (S4) des données de commande afin de commander le laser chirurgical ophtalmique (18), lesquelles données utilisent la géométrie de déformation pour séparer le lenticule (12),
**caractérisé en ce que** la valeur de correction est déterminée à partir d'un écart de la hauteur de lenticule, qui est attendue en fonction de la géométrie de lenticule, et d'une hauteur de lenticule atteinte, la hauteur de lenticule atteinte étant déterminée après une application du dispositif de traitement (10) sans utiliser la géométrie de déformation.

2. Procédé selon la revendication 1, **caractérisé en ce que** la valeur de correction est déterminée statistiquement à partir de plusieurs mesures précédentes de la hauteur de lenticule et de la hauteur de lenticule atteinte, en particulier au moyen d'une régression linéaire (LR).

3. Procédé selon l'une des revendications précédentes, **caractérisé en ce que** la hauteur de lenticule atteinte est déterminée à au moins deux instants différents, la valeur de correction étant déterminée à partir d'une valeur moyenne des écarts aux au moins deux instants différents.

4. Procédé selon l'une des revendications précédentes, **caractérisé en ce que** la valeur de correction est combinée avec une valeur de correction de valeur de réfringence, la valeur de correction de valeur de réfringence étant déterminée à partir d'un écart de la valeur de réfringence à corriger et d'une valeur de réfringence atteinte, en particulier au moyen d'une régression linéaire à partir de plusieurs mesures précédentes et d'une valeur moyenne d'écarts de valeur de réfringence à différents instants, la valeur de réfringence atteinte étant déterminée après une application du dispositif de traitement (10).

5. Procédé selon l'une des revendications précédentes, **caractérisé en ce que** la valeur de correction est dans une plage de 0,7 à 1,5, en particulier dans une plage de 1,0 à 1,4.

6. Procédé selon l'une des revendications précédentes, **caractérisé en ce que** le diamètre de déformation est calculé au moyen d'une multiplication du diamètre de lenticule par un paramètre dépendant de la valeur de correction, le paramètre étant établi au moyen d'un modèle de géométrie théorique de lenticule, en particulier au moyen de la formule de Munnerlyn.

7. Procédé selon la revendication 6, **caractérisé en ce qu'**une hauteur est déterminée pour la hauteur de déformation de la géométrie de déformation, laquelle hauteur correspond à une multiplication de la hauteur de lenticule par la valeur inverse de la valeur de correction, le diamètre de déformation étant calculé au moyen d'une division du diamètre de lenticule par la racine carré de la valeur de correction.

8. Dispositif de commande (20) configuré pour mettre en oeuvre un procédé selon l'une des revendications précédentes.

9. Dispositif de traitement (10) comportant au moins un laser chirurgical ophtalmique (18) pour la séparation d'un lenticule (12) d'un oeil humain ou animal (36) au moyen d'une photodisruption, et au moins un dispositif de commande (20) selon la revendication 8.

10. Dispositif de traitement selon (10) la revendication 9, **caractérisé en ce que** le laser (18) est conçu pour émettre des impulsions laser dans une gamme de longueurs d'onde entre 300 nm et 1 400 nm, de préférence entre 700 nm et 1 200 nm, à une durée d'impulsion respective entre 1 fs et 1 ns, de préférence entre 10 fs et 10 ps, et à une fréquence de répétition supérieure à 10 kHz, de préférence entre 100 kHz et 100 MHz.

11. Dispositif de traitement (10) selon l'une des revendications 9 ou 10, **caractérisé en ce que** le dispositif de commande (20)
- comporte au moins un dispositif de stockage pour le stockage au moins temporaire d'au moins un ensemble de données de commande, l'ensemble ou les ensembles de données de commande comprenant des données de commande pour le positionnement et/ou la focalisation d'impulsions laser individuelles dans la cornée ; et
- au moins un dispositif à faisceau (22) pour le guidage de faisceau et/ou la conformation de faisceau et/ou la déviation de faisceau et/ou la focalisation de faisceau d'un faisceau laser (24) du laser (18).

12. Programme informatique comprenant des instructions qui amènent le dispositif de traitement (10) selon l'une des revendication 9 à 11 à mettre en oeuvre un procédé selon l'une des revendications 1 à 7.

13. Support lisible par ordinateur sur lequel est stocké le programme informatique selon la revendication 12.
